# EUROPÄISCHE PATENTSCHRIFT

(11) **EP 2 748 590 B1**
(45) Veröffentlichungstag und Bekanntmachung des Hinweises auf die Patenterteilung: **10.05.2017**
(21) Anmeldenummer: 12748432.7
(22) Anmeldetag: 15.08.2012
(51) Int. Cl.: G01N 23/00, G01V 5/00, G01N 23/02, G01T 7/00, G01N 33/22

(54) **FLÜSSIGES GEMISCH ZUM TESTEN UND VALIDIEREN VON PRÜFGERÄTEN ZUR ÜBERPRÜFUNG VON GEGENSTÄNDEN ODER PERSONEN**
LIQUID MIXTURE USED TO TEST AND VALIDATE TEST DEVICES FOR INSPECTING OBJECTS OR PERSONS
MÉLANGE LIQUIDE POUR TESTER ET VALIDER DES APPAREILS DE CONTRÔLE DESTINÉS À CONTRÔLER DES OBJETS OU DES PERSONNES

(30) Priorität: 22.08.2011 DE 102011081328
(43) Veröffentlichungstag der Anmeldung: 02.07.2014
(73) Patentinhaber: Smiths Heimann GmbH, 65205 Wiesbaden (DE)
(72) Erfinder: SIEDENBURG, Uwe, 55270 Essenheim (DE)
(74) Vertreter: Greif, Thomas
(86) Internationale Anmeldenummer: PCT/EP2012/065948
(87) Internationale Veröffentlichungsnummer: WO 2013/026751

(56) Entgegenhaltungen:
- WO-A1-2005/094768
- US-A- 2 495 277

## Beschreibung

Zur Überprüfung von Personen und Objekten, wie Gepäckstücken, auf gefährliche Stoffe, wie Spreng- oder Explosivstoffe, werden Prüfanlagen verwendet, in denen die zu prüfenden Personen oder Objekte in Prüfgeräten von elektromagnetischen Strahlen durchleuchtet oder bestrahlt werden. Derartige Prüfanlagen werden an Flughäfen zur Überprüfung von Passagieren und Gepäckstücken eingesetzt.

Die Prüfanlagen enthalten nach einer bekannten Ausführungsform Prüfgeräte, bei denen die zu überprüfenden Objekte, beispielsweise die Gepäckstücke, von Röntgenstrahlen durchleuchtet oder bestrahlt werden und die transmittierten oder gestreuten Röntgenstrahlen detektiert und ausgewertet werden (DE 10125531-A, DE 19954662-A).

Zur Überprüfung von Personen sind Prüfgeräte bekannt, bei denen die zu überprüfenden Personen mit elektromagnetischen mm-Wellen bestrahlt und die gestreuten mm-Wellen für eine bildhafte Darstellung ausgewertet werden (DE 102005016106-A).

Vor der Inbetriebnahme müssen die Prüfgeräte getestet und validiert werden. Dies erfolgt üblicherweise mit den realen Gefahrstoffen, also den zu detektierenden Sprengstoffen. Die Benutzung von Sprengstoffen ist gesetzlich geregelt, zudem sind sie schwer handhabbar.

Aus dem US-Patent 5,958,299 ist ein Sprengstoff-Simulationsgemisch bekannt, das nicht explosive Bestandteile enthält, wobei die Komponenten so ausgewählt sind, dass die Mischung eine physikalische Form, Dichte, Röntgentransmission und eine effektive Ordnungszahl aufweist, die einem ausgewählten Sprengstoffgemisch entspricht. Mit dem Simulationsgemisch anstelle eines realen Sprengstoffes lässt sich ein Röntgenprüfgerät auf die Detektion des bestimmten Sprengstoffes testen. Als Simulationsgemische sind feste, plastische und gelförmige Zusammensetzungen beschrieben, die sich aus verschiedenen Komponenten aufbauen.

Die WO 2005/094768 A1 offenbart eine Mischung, die Glycerin und Natriumhydroxid in einem Gewichtsverhältnis von Glycerin zu Natriumhydroxid = 6,25 (20/3,2) enthält.

Die US 2,495,277 A offenbart eine Mischung, die aus 20% Wasser, 15% Natriumhydroxid und 65% Glycerin besteht.

An Prüfanlagen wird zunehmend die Anforderung gestellt, auch Flüssigkeitssprengstoffe und sogenannte "home made explosives" zu detektieren. Der Erfindung liegt daher die Aufgabe zugrunde, eine derartige Spreng- oder Explosivstoffe simulierende Materialmischung bereitzustellen, die nicht explosiv, unkritisch in der Handhabung und preisgünstig in der Herstellung ist, sowie sich in einem Prüfgerät zur Überprüfung von Gegenständen oder Personen verhält wie der reale Gefahrstoff.

Diese Aufgabe wird mit einem Gemisch aus Glycerin, Natriumhydroxid (NaOH) und Wasser gelöst, wobei Glycerin und Natriumhydroxid in einem Gewichtsverhältnis Glycerin / Natriumhydroxid zwischen 6,5 und 3,8 vorliegen.

Bei einem bevorzugten Simulationsgemisch beträgt der Anteil von Glycerin 57 Gew.-% - 78 Gew.-%, insbesondere ca. 68 Gew.-%, der Anteil von Natriumhydroxid 12 Gew.-% - 15 Gew.-%, insbesondere ca. 14 Gew.-%, wobei der Anteil von Wasser die Anteile zu 100 Gew.-% ergänzt, insbesondere ca. 18 Gew.-% beträgt.

Die Erfindung bietet so die Möglichkeit, auch explosive Flüssigkeiten zu simulieren. Durch unterschiedliche Verdünnungsgrade mit Wasser lassen sich Simulationsgemische für verschiedene flüssige Gefahrstoffe herstellen.

Die Erfindung wird zum Testen und Validieren von Prüfgeräten verwendet, die in der Lage sind, flüssige Gefahrstoffe zu detektieren. Dazu werden die zu überprüfenden Gegenstände, insbesondere Gepäckstücke, mit elektromagnetischen Strahlen durchleuchtet oder bestrahlt. Die transmittierten oder gestreuten Strahlen werden detektiert oder empfangen und ausgewertet. Die Prüfgeräte müssen zur Sicherung ihrer Funktionsfähigkeit getestet und validiert werden. Bei der Validation wird geprüft, ob die Prüfanlage vorbestimmte Anforderungen erfüllt.

Für den Test und die Validation wird in einem zu überprüfenden Gegenstand oder an einer Person das flüssige Simulationsgemisch nach der Erfindung angeordnet. Das Simulationsgemisch besteht aus Glycerin, Natriumhydroxid und Wasser.

Eine bevorzugte Verwendung eines Gemischs nach der Erfindung erfolgt zum Testen und Validieren von Röntgenprüfgeräten zur Überprüfung von Personen oder Gegenständen, insbesondere von Gepäckstücken, wie sie in der Beschreibungseinleitung als bekannt beschrieben werden.

Eine weitere bevorzugte Verwendung eines Gemischs nach der Erfindung erfolgt zum Testen und Validieren von Prüfgeräten, die elektromagnetische mm-Wellen verwenden, zur Überprüfung von Personen oder Gegenständen, wie sie ebenfalls in der Beschreibungseinleitung als bekannt beschrieben werden.

Nach der Grundrezeptur für das flüssige Simulationsgemisch liegen Glycerin und Natriumhydroxid (NaOH) in einem Gewichtsverhältnis Glycerin / Natrium zwischen 6,5 und 3,8 vor. Es ist Wasser zugesetzt, das die Mischung aus Glycerin und NaOH auf 100 Gew.-% ergänzt. Bevorzugt beträgt der Anteil von Glycerin 57 Gew.-% bis 78 Gew.-%, insbesondere ca. 68 Gew.-% und der Anteil von Natriumhydroxid (NaOH) 12 Gew.-% bis 15 Gew.-%, insbesondere ca. 14 Gew.-%. Die Mischung von Glycerin und Natriumhydroxid (NaOH) wird durch Wasser auf 100 Gew.-% ergänzt wird, wobei der Wasseranteil bevorzugt ca. 18 Gew.-% beträgt.

Die vorstehend beschriebene Grundrezeptur lässt sich unter Beibehaltung des Verhältnisses zwischen Glycerin und Natriumhydroxid durch mehr oder weniger Wasser verdünnen oder verdicken, wobei das Gesamtgemisch flüssig bleibt. Durch die unterschiedlichen Anteile von Wasser lassen sich Simulationsgemische zur Simulation von verschiedenen flüssigen Gefahrstoffen herstellen.

## Patentansprüche

1. Verwendung eines Gemischs enthaltend Glycerin und Natriumhydroxid in einem Gewichtsverhältnis Glycerin / Natriumhydroxid zwischen 6,5 und 3,8 und Wasser zum Testen und Validieren von Röntgenprüfgeräten und Prüfgeräten, die elektromagnetische mm-Wellen verwenden, zur Überprüfung von Personen oder Gegenständen, insbesondere von Gepäckstücken.

2. Verwendung eines Gemischs nach Patentanspruch 1, wobei der Anteil von Glycerin 57 Gew.-% - 78 Gew.-% und der Anteil von Natriumhydroxid 12 Gew.-% - 15 Gew.-%, insbesondere ca. 14 Gew.-% beträgt, wobei die Mischung von Glycerin und Natriumhydroxid durch Wasser auf 100 Gew.-% ergänzt wird und der Wasseranteil bevorzugt ca. 18 Gew.-% beträgt.

## Claims

1. Use of a mixture containing glycerol and sodium hydroxide in a weight ratio of glycerol/sodium hydroxide between 6.5 and 3.8 and water for testing and validating x-ray screening devices and screening devices which use electromagnetic mm waves for inspecting persons or objects, particularly luggage.

2. Use of a mixture according to Claim 1, wherein the proportion of glycerol is 57 wt% - 78 wt% and the proportion of sodium hydroxide is 12 wt% - 15 wt%, particularly about 14 wt%, wherein the mixture of glycerol and sodium hydroxide is complemented with water to 100 wt% and the proportion of water is preferably about 18 wt%.

## Revendications

1. Utilisation d'un mélange contenant du glycérol et de l'hydroxyde de sodium, dans un rapport pondéral glycérol/hydroxyde de sodium entre 6,5 et 3,8, et de l'eau pour tester et valider des appareils de test aux rayons X et des appareils de test qui utilisent des ondes mm électromagnétiques, pour examiner des personnes ou des objets, en particulier des bagages.

2. Utilisation d'un mélange selon la revendication 1, la proportion de glycérol étant de 57% en poids - 78% en poids et la proportion d'hydroxyde de sodium étant de 12% en poids - 15% en poids, en particulier d'environ 14% en poids, le mélange de glycérol et d'hydroxyde de sodium étant complété par de l'eau à 100% en poids et la proportion d'eau étant de préférence d'environ 18% en poids.
